(19) **Europäisches Patentamt**
**European Patent Office**
**Office européen des brevets**

(11) **EP 4 123 589 A1**

(12) **EUROPEAN PATENT APPLICATION**

(43) Date of publication:
**25.01.2023 Bulletin 2023/04**

(21) Application number: **21186782.5**

(22) Date of filing: **20.07.2021**

(51) International Patent Classification (IPC):
**G06T 19/20** (2011.01)

(52) Cooperative Patent Classification (CPC):
**G06T 19/20; A61B 8/523; G06T 7/12; G06T 7/149;**
G06T 2207/10088; G06T 2207/20101;
G06T 2207/30048; G06T 2207/30081;
G06T 2210/41; G06T 2219/2021

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **WEBER, Frank Michael**
  **5656 AE Eindhoven (NL)**
• **PETERS, Jochen**
  **5656 AE Eindhoven (NL)**
• **WEESE, Juergen**
  **5656 AE Eindhoven (NL)**

(74) Representative: **Philips Intellectual Property &
Standards**
**High Tech Campus 52**
**5656 AG Eindhoven (NL)**

(54) **METHOD AND SYSTEM FOR IMAGE PROCESSING**

(57) According to an aspect, there is provided a computer implemented method for determining editing to be applied to a three-dimensional, 3D, mesh, wherein the 3D mesh represents a segmentation of a 3D image, the method comprising, responsive to receiving an indication of an adjustment to be applied to a first position on the 3D mesh in an editing plane of the 3D image, adjusting a point on a boundary of the 3D mesh in an editing region of the 3D mesh using an anisotropic weighting factor that restricts the adjustment made to the point if the point lies in a first direction relative to the editing plane.

FIG. 2

EP 4 123 589 A1

**Description**

FIELD OF THE INVENTION

The invention relates to image processing, and more particularly, image processing for determining editing to be applied to a three-dimensional, 3D, mesh.

BACKGROUND OF THE INVENTION

This disclosure lies in the field of 3D mesh manipulation of a 3D mesh which has been generated as a result of segmentation of an image. The disclosures herein may be applied to a diverse range of images, such as for example, medical images. Image segmentation involves extracting shape/form information about the objects or shapes captured in an image. This may be achieved by converting the image into constituent blocks or "segments" that represent the different features in the image. Image segmentation may comprise fitting a model to one or more features in an image.

One method of image segmentation is Model-Based Segmentation (MBS), whereby a triangulated three dimensional (3D) mesh of a target structure (such as, for example, a heart, brain, lung etc.) is adapted in an iterative fashion to features in an image. Segmentation models typically encode population-based appearance features and shape information. Such information describes permitted shape variations based on real-life shapes of the target structure in members of the population. Shape variations may be encoded, for example, in the form of Eigenmodes which describe the manner in which changes to one part of a model are constrained, or dependent, on the shapes of other parts of a model.

Model-based segmentation has been used in various applications to segment one or multiple target organs from medical images, see, for example, the paper by Ecabert, O., et al. 2008 entitled "Automatic Model-Based Segmentation of the Heart in CT Images"; IEEE Trans. Med. Imaging 27 (9), 1189-1201. The use of triangulated surface meshes has led to MBS generally providing smooth segmentation results. Furthermore, MBS is generally considered to be robust against image artifacts, such as variations in image quality.

Interactive 3D mesh editing is typically used to manually fine-tune automatic segmentation results such as those described above. Once a model comprising a 3D mesh has been produced, the 3D mesh is displayed as an overlay on an image, for example the image which was used as the basis for segmentation. A user is able to edit the mesh by dragging the mesh region to be edited to a desired target location in order that the 3D mesh better conforms to the object in the image represented by the 3D mesh. The editing of the mesh is propagated out to other points on the mesh from the point at which the mesh has been edited, and is typically constrained to a certain proportion of the mesh from the point at which the mesh is edited. This may be referred to as an editing region, or an influence region. It is desirable to improve the way in which the editing region is defined to enable efficient editing of a 3D mesh.

SUMMARY OF THE INVENTION

A 3D volume acquired using MR (magnetic resonance) typically has an anisotropic resolution due to the acquisition process. A 3D volume resulting from MR analysis often has one particular axis that has a very different resolution to the other axes. As an MR image is acquired slice-by-slice, often the full temporal information is acquired for each slice sequentially, which may lead to mismatch between slices due to changes in the region of a subject corresponding to the slices that occurs over time.

A 3D mesh may be generated for a 3D image, such as an MR (magnetic resonance) image made up of a plurality of slices, using a segmentation method such as those described above. A user may then edit the 3D mesh so that the mesh better conforms to the object that the 3D mesh represents. A problem for 3D editing tools in MR applications is that only slice-by-slice editing of the 3D mesh may be available in the user interface. In cardiac MR, this means that often only short-axis slices are being edited. 3D mesh editing tools typically use an influence region that isotropically decays along all space dimensions. A small editing region can restrict editing to the slice, but does not allow larger edits within the slice. A large editing region can provide a large editing region within the slice, but also affects neighbour slices, which may not be desired.

It has been determined by the inventors that an improved configuration of an editing region would be one in which large regions within the slice are affected, but the impact on neighbour slices can be restricted. It is an objective of embodiments herein to provide an improved method for determining editing to be applied to a 3D mesh.

Thus, according to an aspect there is provided a computer implemented method for determining editing to be applied to a three-dimensional, 3D, mesh, wherein the 3D mesh represents a segmentation of a 3D image, the method comprising: responsive to receiving an indication of an adjustment to be applied to a first position on the 3D mesh in an editing plane of the 3D image, adjusting a point on a boundary of the 3D mesh in an editing region of the 3D mesh using an anisotropic weighting factor that restricts the adjustment made to the point if the point lies in a first direction relative to the editing plane. This method may be applicable to images obtained using various imaging methods, such as MR, which typically constructs an image by acquiring slices of images, or in ultrasound, where editing may be performed by altering the 3D mesh by interacting with reformatted planes of an ultrasound image.

It is assumed herein that that any standard processes may be used in addition to the anisotropic weighting factor to determine the adjustment to be made to the point. For example the anisotropic weighting factor may be applied to an editing determined for the point based on a propagation of the adjustment of the first position to the point on the boundary of the 3D mesh. The editing may be determined using any conventional 3D mesh editing processes.

The point may be a vertex of the 3D mesh. The 3D mesh may define a boundary. Vertices of the 3D mesh may define points on the boundary of the 3D mesh. The method may be applied to a plurality of points on the boundary of the 3D mesh using an anisotropic weighting factor which differs depending on the distance of a particular point from a plane (e.g. the editing plane). The anisotropic weighting factor may restrict the adjustment made to the point in the first direction.

The invention therefore relates to the restriction of editing of a 3D mesh for a portion of the mesh lying in a first direction relative to the editing plane using an anisotropic weighting factor. This advantageously enables directional restriction of an editing region. The anisotropic (e.g. directional) weighting factor may restrict editing in a particular direction, for example the first direction. The anisotropic weighting factor may restrict a component of the editing in a particular direction, which may be the first direction. In particular, the editing of a 3D mesh may be restricted in a direction which is in a direction normal to the editing plane. The editing plane may be comprised in a slice of the 3D image, and may be a central plane of the slice. This may allow, for example, editing of the 3D mesh in slices which have not yet been manipulated by a user, but preventing (or substantially reducing) editing of slices which have already been manipulated by a user. For example, the 3D image may comprise a plurality of slices to be edited in succession, and the first direction may oppose a direction in which slices are to be edited. In particular, editing of the slice adjacent to the slice comprising the editing plane may be restricted such that zero adjustment is applied to a central editing plane of the slice adjacent to the slice comprising the editing plane. Furthermore, this may allow a large region within the slice to be edited. The method may be beneficial for images which have anisotropic resolution (for example, cardiac MR or prostate MR), as these images may have a high resolution in one direction but a low resolution in another direction. Therefore, editing in the direction in which there is low resolution may be restricted but the editing in the direction in which there is higher resolution may be less restricted. For example, the resolution of the 3D image may be high in a direction parallel to a plane of the slice, but low in a direction normal to a slice. The editing of the 3D mesh may be restricted in a direction which is parallel to an axis of an anatomical feature in the 3D image. Restricting editing in particular directions (such as the normal to a slice) may enable a smooth transition to be provided between edited regions and frozen regions (e.g. regions which are not to be edited).

Editing of the 3D mesh may be restricted in a first and second direction normal to the editing plane (e.g.

directions opposing one another and normal to the editing plane). In particular, the editing of the mesh may be restricted in a first direction (e.g. in a direction normal to and above the editing plane) for a point of the mesh which lies in the first direction, and the editing of the mesh may be restricted in a second direction (e.g. in a direction normal to and below the editing plane) for a point of the mesh which lies in the second direction. Thus, the editing of the mesh may be restricted so that in a first and second direction normal to the editing plane, the editing is restricted (e.g. to within the slice or such that a central plane of an adjacent slice is not edited) but in directions parallel to the editing plane, the editing is not restricted by the anisotropic weighting function. Therefore, a large region within a slice may be edited. The first direction may be any of a direction normal to the editing plane; a direction parallel to an axis of interest; a direction parallel to an axis of an anatomical feature in the 3D image; a direction parallel to an axis of a ventricle in the 3D image; a direction parallel to an axis of a prostate in the 3D image, for example.

The anisotropic weighting factor may be determined for the point based on a restriction function. In particular, the anisotropic weighting factor may depend on the distance of the point from a plane (for example the editing plane). The editing region may be a region in which the adjustment applied to the first position on the 3D mesh results in editing of other points on the 3D mesh which are within the editing region by propagation of the editing to other points. The editing region may be defined by the adjustment applied to the first position. The editing region may be a predetermined distance from the indicated adjustment to be applied to the first position. The editing region may be initially defined by an isotropic weighting factor. A restricted editing region may be defined by the restriction function (e.g. the restriction function may restrict editing of points within the editing region). The restricted editing region may represent a region in which editing of the point is not restricted to zero by the anisotropic weighting factor. The restricted editing region of the editing may decay quickly along the given direction (for example, the first direction). In the other directions (for example, parallel to the editing plane), the editing region decay less quickly. To provide a consistent user experience for varying slice thickness, the width and edge sharpness (or smoothness) of the borders or boundaries of the restricted editing region along the slice normal may be defined relative to the slice thickness. Furthermore, the direction in which the editing is restricted may be in only one direction or may be in more than one direction. A different anisotropic weighting factor may be applied to different directions or the same anisotropic weighting factor may be applied to more than one direction. For example, different parameters relating to smoothness and the location of the centre of the boundary of the restricted editing region may be used to define different anisotropic weighting factors for different directions. The direction in which editing is to be restricted may be input by a user. The anisotropic weighting factor may be derived from a smooth weighting function and/or a sigmoid function.

According to a further aspect there is provided a system for determining editing to be applied to a three-dimensional, 3D, mesh, wherein the 3D mesh represents a segmentation of a 3D image, the system comprising: a memory comprising instruction data representing a set of instructions; and a processor configured to communicate with the memory and to execute the set of instructions, wherein the set of instructions, when executed by the processor, cause the processor to: responsive to receiving an indication of an adjustment to be applied to a first position on the 3D mesh in an editing plane of the 3D image, adjusting a point on a boundary of the 3D mesh in an editing region of the 3D mesh using an anisotropic weighting factor that restricts the adjustment made to the point if the point lies in a first direction relative to the editing plane.

According to a further aspect there is provided a computer program product comprising a computer readable medium, the computer readable medium having computer readable code embodied therein, the computer readable code being configured such that, on execution by a suitable computer or processor, the computer or processor is caused to perform any of the methods described herein.

These and other aspects will be apparent from and elucidated with reference to the embodiment(s) described hereinafter.

BRIEF DESCRIPTION OF THE DRAWINGS

Example embodiments will now be described, by way of example only, with reference to the following drawings, in which:

> Fig. 1 is an apparatus for use with the methods described herein according to an example;
> Fig. 2 is a method according to an example;
> Fig. 3 is an example of an editing plane of a 3D image;
> Fig. 4 is a 2D representation of an example of a stack of slices of a 3D image with a 2D representation of a 3D mesh overlaid on the image;
> Fig. 5 is an example of a 2D representation of a 3D image comprising a plurality of slices;
> Fig. 6 shows two graphs illustrating a restriction function where in Fig. 6(a) the effect of the value of the centre of the boundary of the editing region $\mu$ is illustrated, and in Fig. 6(b), the effect of the value of a smoothness parameter $\sigma$ is illustrated;
> Fig. 7 shows two graphs illustrating the restriction function, where in Fig. 7(a) single- and two-sided restriction functions are shown, and in Fig. 7(b) the effect of altering the values of the centre of the boundary of the editing region and the smoothness parameter on a two sided restriction function is shown.

DETAILED DESCRIPTION OF EMBODIMENTS

As described briefly above, editing regions of a 3D mesh are typically isotropic, however, the editing regions may be either too large, and affect neighbouring slices, or too small, and do not affect enough of a slice.

It is an object of embodiments herein to provide methods and systems for determining editing to be applied to a 3D mesh. The 3D mesh may have been obtained by segmenting a 3D image obtained using a conventional imaging system such as an MRI or ultrasound system.

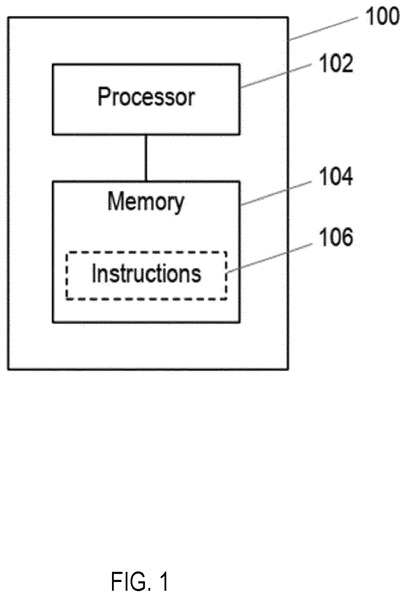

Turning now to Fig. 1 in some embodiments there is an apparatus 100 for use in determining editing to be applied to a three-dimensional, 3D, mesh, wherein the 3D mesh represents a segmentation of a 3D image, according to some embodiments herein. Generally, the apparatus may form part of a computer apparatus or system e.g. such as a laptop, desktop computer or other computing device. In some embodiments, the apparatus 100 may form part of a distributed computing arrangement or the cloud.

The apparatus comprises a memory 104 comprising instruction data representing a set of instructions and a processor 102 (e.g. processing circuitry or logic) configured to communicate with the memory and to execute the set of instructions. Generally, the set of instructions, when executed by the processor, may cause the processor to perform any of the embodiments of the method for determining editing to be applied to a three-dimensional, 3D, mesh, as described below.

Embodiments of the apparatus 100 may be for use in a system for determining editing to be applied to a three-dimensional, 3D, mesh. More specifically, the set of instructions, when executed by the processor, cause the processor to: responsive to receiving an indication of an adjustment to be applied to a first position on the 3D mesh in an editing plane of the 3D image, adjusting a point on a boundary of the 3D mesh in an editing region of the 3D mesh using an anisotropic weighting factor that restricts the adjustment made to the point if the point lies in a first direction relative to the editing plane.

The processor 102 can comprise one or more processors, processing units, multi-core processors or modules that are configured or programmed to control the apparatus 100 in the manner described herein. In particular implementations, the processor 102 can comprise a plurality of software and/or hardware modules that are each configured to perform, or are for performing, individual or multiple steps of the method described herein. The processor 102 can comprise one or more processors, processing units, multi-core processors and/or modules that are configured or programmed to control the apparatus 100 in the manner described herein. In some implementations, for example, the processor 102 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 104 is configured to store program code that can be executed by the processor 102 to perform the method described herein. Alternatively or in addition, one or more memories 104 may be external to (i.e. separate to or remote from) the apparatus 100. For example, one or more memories 104 may be part of another device. Memory 104 can be used to store, for example, the 3D mesh, vertices and/or points on the boundary of the 3D mesh, editing of points on the boundary of the 3D mesh, images, for example a 3D image, an editing plane of the 3D image, a plurality of slices of a 3D image, data relating to an image, data relating to the anisotropic weighting factor, restriction function, weighting factors, and/or any other information or data received, calculated or determined by the processor 102 of the apparatus 100 or from any interfaces, memories or devices that are external to the apparatus 100. The processor 102 may be configured to control the memory 104 to store the 3D mesh, vertices and/or points on the boundary of the 3D mesh, editing of points on the boundary of the 3D mesh, images, for example a 3D image, an editing plane of the 3D image, a plurality of slices of a 3D image, data relating to an image, data relating to the anisotropic weighting factor, restriction function, and weighting factors.

In some embodiments, the memory 104 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 1 only shows the components required to illustrate this aspect of the disclosure and, in a practical implementation, the apparatus 100 may comprise additional components to those shown. For example, the apparatus 100 may further comprise a display. A display may comprise, for example, a computer screen, and/or a screen on a mobile phone or tablet. The apparatus may further comprise a user input device, or interface, such as a keyboard, mouse or other input device that enables a user to interact with the apparatus, for example, to provide indications of adjustments to be used in the method described herein. The apparatus 100 may comprise a battery or other power supply for powering the apparatus 100 or means for connecting the apparatus 100 to a mains power supply.

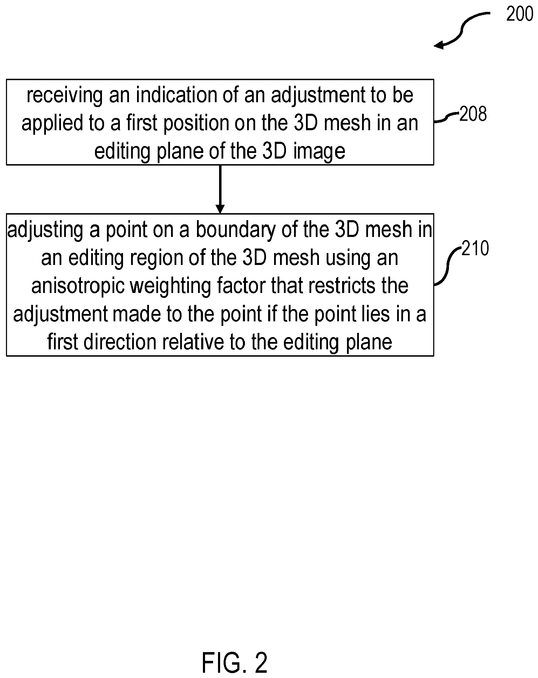

Turning to Fig. 2, there is a computer implemented method 200 for use in dermining editing to be applied to a three-dimensional, 3D, mesh, wherein the 3D mesh represents a segmentation of a 3D image. Embodiments of the method 200 may be performed, for example by an apparatus such as the apparatus 100 described above.

Briefly, the method 200 comprises adjusting a point on a boundary of the 3D mesh in an editing region of the 3D mesh using an anisotropic weighting factor that restricts the adjustment made to the point if the point lies in a first direction relative to the editing plane 210, responsive to receiving an indication of an adjustment to be applied to a first position on the 3D mesh in an editing plane of the 3D image 208.

The received indication may be based on input of a user. The received indication may be an indication of a movement of the 3D mesh from a first position on the 3D mesh to a second position, for example, in order to better conform to the object represented by the 3D mesh. The received indication of an adjustment may be propagated out to other points in the 3D mesh, for example, within the editing region. The anisotropic weighting factor may be applied to the propagated editing in order to restrict the propagated editing, or adjustment to be made to a point on a boundary of the 3D mesh, in a particular direction.

The anisotropic weighting factor may be determined for the point based on a restriction function. The restriction function may be a smooth weighting function and/or a sigmoid function. Parameters of the restriction function may comprise at least one of: a distance of the point from the editing plane in the first direction; a smoothness parameter; the first direction. Thus, the anisotropic weighting factor may differ depending on the distance of a point from the editing plane in the first direction. For example, where a point on the boundary of the 3D mesh is a large distance from the editing plane in the first direction, the restriction function may tend to zero. Where a point on the boundary of the 3D mesh is very close to the editing plane in the first direction, the restriction function may tend to one. The adjustment of the point may depend on the received indication of an adjustment, where the anisotropic weighting factor is applied to an adjustment which may have been propagated to the first point from the indication of adjustment of the first position.

It will be appreciated that an isotropic weighting factor may also be applied to the editing of the mesh, which may correspond to the editing region around the first position on a 3D mesh that has been edited by a user. The anisotropic weighting factor may be applied to the editing of a point in addition to the isotropic weighting factor, thereby further restricting editing of the point in the first direction. The restriction function may restrict the editing region to define a restricted editing region. The restricted editing region may be restricted relative to the editing region in at least one of the first direction, the first direction and a second direction opposite to the first direction.

It will be appreciated that the method is not limited to the adjustment of a single point on the boundary of the 3D mesh, but may be applied to a plurality of points on the boundary of the 3D mesh, where any of the plurality of points that lie in the first direction relative to the editing plane may be adjusted using an anisotropic weighting factor (for example, any points that lie above the editing plane). The anisotropic weighting factor applied may differ depending on the distance of a point from a plane (e.g. the editing plane). For example, the restriction function may take as input the distance of a point from a plane to determine the anisotropic weighting factor to be applied. The method may be applied to a vertex which is a point on the boundary of the 3D mesh, and/or to a plurality of vertices which are points on the boundary of the 3D mesh. The anisotropic weighting factor may be determined for each of the vertices using the restriction function and based on a distance of the vertex from the editing plane in the first direction. Thus, the anisotropic weighting factor may be different for each vertex.

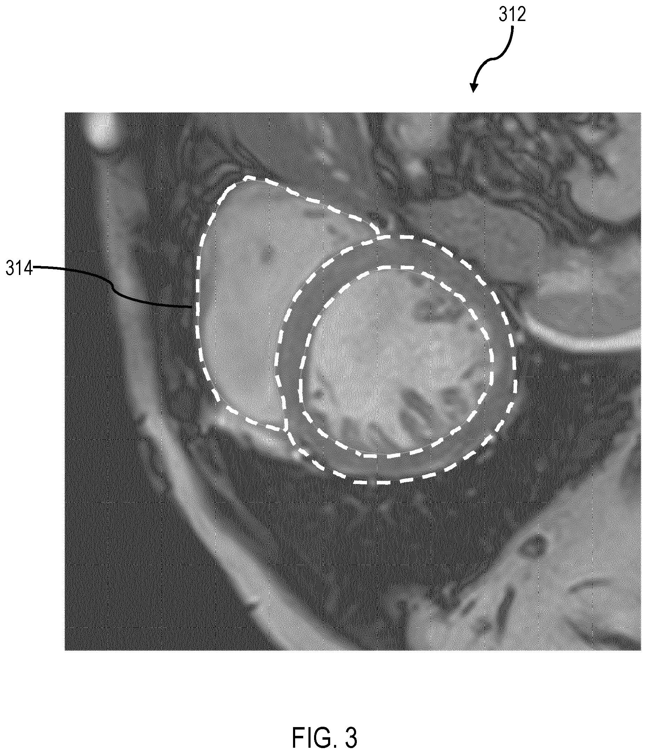

Fig. 3 illustrates an editing plane 312 of a 3D image according to an example. In particular, Fig. 3 illustrates an editing plane comprised in a slice of a 3D image of a heart. Segmentation has been applied to the 3D image to produce a 3D mesh of the heart. A 2D representation of the 3D mesh is illustrated as a dotted line 314 in this Figure. A user may interact with the 3D mesh by clicking and dragging directly on the image (in this example, clicking and dragging on the 2D representation of the 3D mesh 314).

Typically, in order to edit a 3D mesh, a user first looks at an image with overlaid segmentation result and identifies a region to edit. The user then clicks into the centre of the region to edit and holds down a mouse button. The user then drags the mouse to the desired location, while the displayed mesh is being dynamically deformed, i.e., a region around the start point is shifted towards the new mouse position. The mesh is deformed in 3D space. The user then releases the mouse button at the desired location. The deformed mesh is displayed and may be used for further analysis such as a volume calculation.

As is discussed above, the deformation of the 3D mesh may be initially restricted to an editing region which may be defined as a distance from the centre of the editing region (e.g. the first point at which a user clicked), where typically the editing of the position edited by the user is propagated out to other points within the editing region. The propagation of editing may decay isotropically. Thus, a weighting may be applied to the editing of vertices or points on the boundary of the 3D mesh within the editing region based on the distance of a point from the centre of the editing region. The weighting may be between 0 and 1, where the further away the point is from the edited point, the closer the weighting gets to a value of 0.

According to examples herein, for points on the boundary of the 3D mesh (for example each vertex of the 3D mesh), an anisotropic weighting factor $k_{rest}$ between 0 and 1 that determines which fraction of the otherwise used editing should be applied to the editing of a point on the boundary of the 3D mesh to restrict editing in a particular direction (which may be the first direction) may be determined. Thus, the component of editing of a point in a direction parallel to the editing plane may have a weighting of 1, whereas a component of editing of a point in a direction normal to the editing plane may have a weighting between 0 and 1.The anisotropic weighting factor may be applied to points within an editing region.

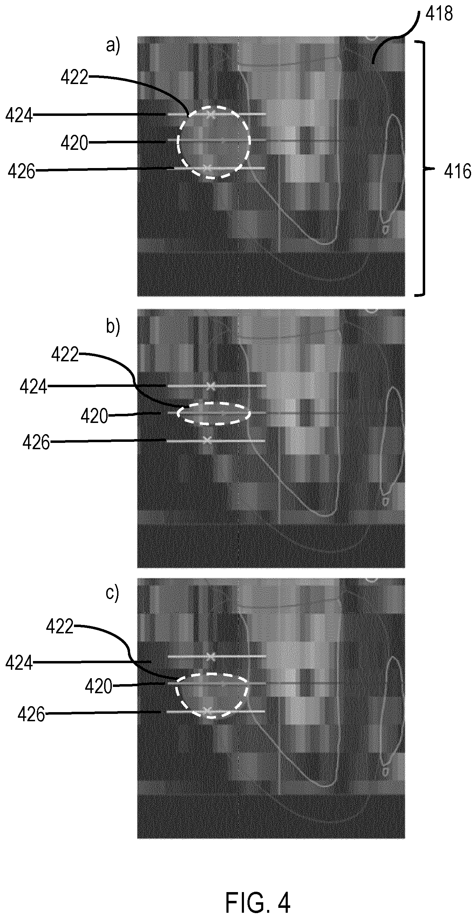

Fig. 4 (a)-(c) illustrates a stack of a plurality of slices of a 3D image 416, viewed as a 2D plane through the stack. In this example, the image shows a heart of a subject, however, the method may be applicable to any part of a subject. Segmentation has been applied so that a 3D mesh representing the structure of the heart has been produced. In this image, the 3D mesh is illustrated as a 2D representation 418. In each of Figs. 4 (a)-(c) the user has edited the mesh in an editing plane of an editing slice 420 (the editing occurs in the 2D image of the editing plane such as that illustrated in Fig. 3). Fig. 4 (a) illustrates a typical editing region 422 resulting from the editing of a user which is shown as a circular region. It will be appreciated that the dotted line in this image represents the border of an editing region, however, it will be further appreciated that this line is merely illustrative of the border, and the editing region may have a smooth border which fades gradually. Points on the boundary of the 3D mesh that are within this region will be edited in response to the deformation of the 3D mesh. As is shown in this Figure, a typical editing region may overlap with adjacent slices above and below the slice comprising the editing plane 420. In particular, the editing region overlaps with a central plane (e.g. an editing plane) of each of the two adjacent slices 424, 426. This may not be desirable if one or both of the adjacent slices have previously been edited by a user, where further editing of an adjacent slice caused by an editing of a current slice may result in a user being required to re-edit the previously edited adjacent slice.

Fig. 4 (b) illustrates an example in which an anisotropic weighting factor (or restriction function) is used according to some examples herein. As is illustrated in Fig. 4(b), the editing region 422 is restricted by the restriction function in a direction normal to the editing plane 420, in two directions (a first direction and a second direction) both "up" and "down" relative to the editing plane. It will be appreciated that the direction being normal to the editing plane is not the only possible direction in which the editing could be restricted, and the direction could be instead in a direction parallel to an axis of interest, such as an axis of a ventricle in the 3D image. The direction of restriction could be chosen anatomically, e.g. based on the segmentation result. As an example, editing could only be allowed above the mitral valve plane, but restricted below. As is shown in this Figure, editing of the 3D mesh is restricted to within the slice in a first and second direction normal to the editing plane. Thus, the editing region does not overlap with central planes of each of the two adjacent slices 424, 426. However, in directions parallel to the editing plane, the editing region 422 is not restricted (relative to the original editing region or the editing region shown in Fig. 4 (a)).

Fig. 4(c) illustrates a further example in which an anisotropic weighting factor (or restriction function) is used according to some examples herein. In contrast to the example of Fig. 4(b), in this example the restriction function only restricts editing in one direction normal to the editing plane 420 (only an "up" direction). As is shown in this Figure, editing is therefore restricted to within the slice in a first direction normal to the editing plane. The editing region therefore does not overlap with the central plane of a first adjacent slice 424, but does overlap with the central plane of a second adjacent slice 426. This may be desirable in a case where slices in one direction relative to (e.g. above) the editing plane have previously been edited, but slices in another direction relative to the editing plane (e.g below) have not. Typically when reviewing editing planes of slices and making edits to the 3D mesh, a user will process through a stack of slices (usually from top to bottom). Therefore, using the methods described herein, a user may not have to revisit previously edited planes, as they will not change based on further alterations in subsequent slices made by the user. However, this particular configuration enables a larger editing region in the plane of the slice, and in a region extending below the editing plane, which may lead to a quicker conforming of the 3D mesh to its actual position as indicated by a user.

It is noted that in this example, the editing region is restricted to within a slice. However, it will be appreciated that the editing region may be restricted so that editing of an adjacent slice occurs, but only up to a centre plane of an adjacent slice (e.g. an editing plane), where the editing becomes zero. In this way, a centre plane of an adjacent slice (e.g. an editing plane that has previously been edited) is not altered based on the alteration in the editing slice, but a smooth transition of the 3D mesh may be provided between the editing planes of the slices.

Where the 3D image is comprised of a plurality of slices as illustrated in Fig. 4, the anisotropic weighting factor may be determined as explained below. In the example below, the position of a point on a boundary of the 3D mesh is described as a vertex on the 3D mesh. However, it will be appreciated that the invention is not restricted to vertices and the methods may be applied to any point on the 3D mesh.

To describe the position of a vertex *p* relative to the current editing plane along the direction of the slice normal, z, the normalized offset from the current editing plane is determined using equation 1 as follows:

$$z = (p - p_{\text{plane}}) * n_{\text{slice}} / \Delta_{\text{slice}} \qquad (1)$$

where $p_{\text{plane}}$ is an arbitrary point in the editing plane (typically the centre plane of the slice), $n_{\text{slice}}$ is the slice normal, and $\Delta_{\text{slice}}$ is the offset between two slice centres.

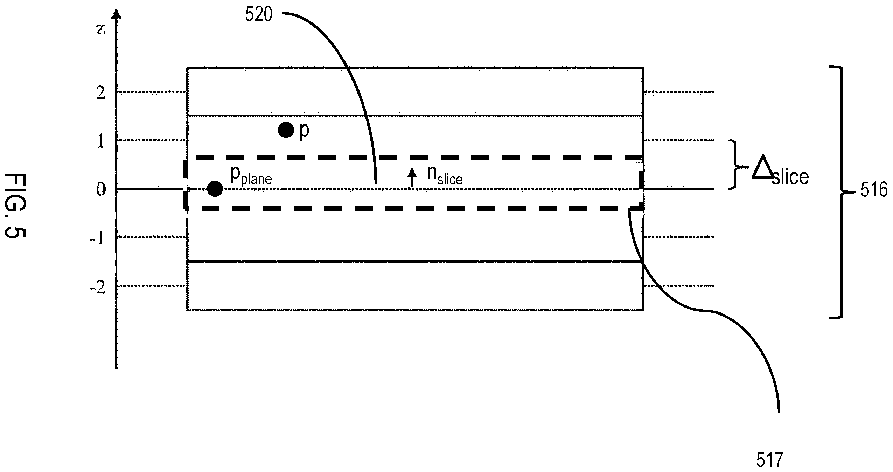

Fig. 5 illustrates a 2D representation of a 3D image comprising plurality of slices 516 such as those described in relation to Fig. 4. An editing slice 517 is illustrated by a rectangle drawn in dotted lines, and the editing slice comprises an editing plane 520 drawn as a dotted line. The editing plane is the centre plane of the editing slice. The axis "Z" of Fig. 5 describes the distance from the centre of the editing slice, and is normalised with respect to the thickness of a slice, or the offset between two slice centres, $\Delta_{\text{slice}}$. Each slice may have a substantial thickness $\Delta_{\text{slice}}$ of around 6 to 10mm.

The plane offset is normalized (e.g. by dividing the offset by the thickness of a slice $\Delta_{\text{slice}}$) so that the distance between the centre plane of each slice and the centre plane of an adjacent slice has a value of 1. The editing plane is set as 0 offset (z = 0). For a point *p*, z is calculated using equation 1. For *p* shown in Fig. 5, z ~ 1.2.

For points in the editing plane, z is 0. Points at an offset a distance of one slice thickness along the slice normal have z=1, and for the opposite direction along the normal z=-1. Normalizing the slice offset with respect to the thickness of a slice advantageously enables geometrical parameters to be defined independently of the slice thickness. By normalizing the slice offset it may be possible for the properties of the mesh editing dependent on the resolution and orientation of the image coordinate system.

Parameters which account for the width of a boundary of the restricted editing region (in which the anisotropic weighting factor restricts the adjustment made to the vertex but does not restrict the adjustment to zero), and the smoothness of transition between 1 and 0 at the boundary, may be included to determine editing to be applied to a vertex. These parameters may be adjusted based on a current slice offset which has not been normalized. Alternatively, the width and sharpness (e.g. smoothness) of a border of the restricted editing region in the first direction may be defined relative to the thickness of a slice comprising the editing plane. With a normalized plane offset, the parameters may be chosen on a more abstract level, such that a fixed set of parameters produces comparable results for different slice offsets.

To describe the decay of the anisotropic weighting factor $k_{\text{rest}}$, a sigmoid function u which varies from 1 to 0 may be used (see equation 2 below) that enables the centre point of the boundary of the editing region and the smoothness of the transition in the boundary to be selected:

$$u_{\mu,\sigma}(z) = \frac{1}{1 + e^{(z-\mu)/\sigma}} \qquad (2)$$

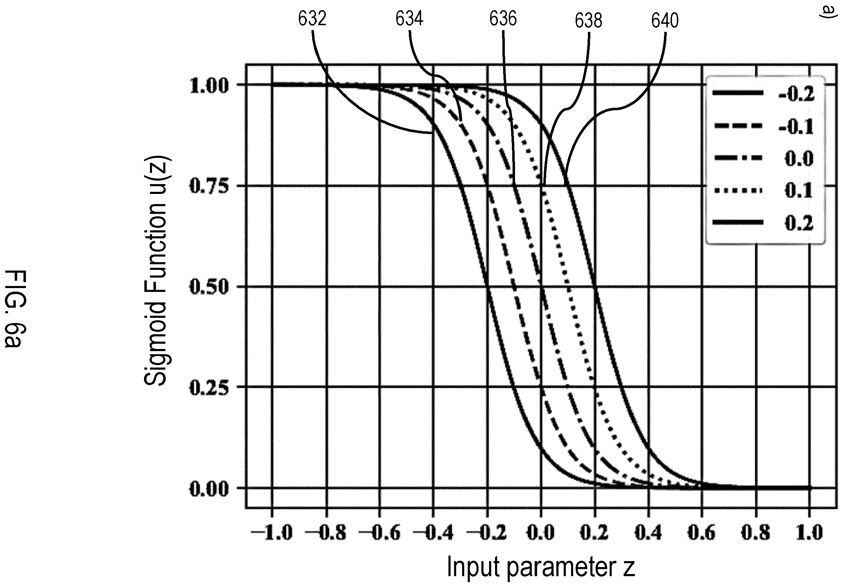

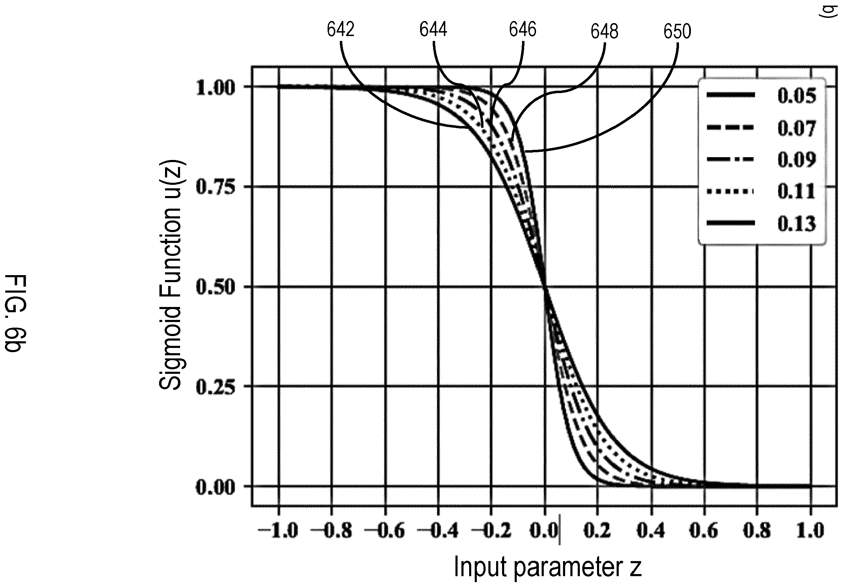

Fig. 6 illustrates, in 6 (a), the sigmoid function as a function of z, where the influence of the value of the centre of the boundary of the editing region $\mu$ on the sigmoid function is illustrated. In this example, values of -0.2, -0.1, 0.0, 0.1 and 0.2 (corresponding to reference numerals 632, 634, 636, 638 and 640 respectively) are set as the values of $\mu$ (where lines for u(z) with these

values of $\mu$ are illustrated in this Figure), and the value of $\sigma$ is set at a constant value. As is illustrated in this Figure, altering the value of the centre of the boundary of the editing region translates the boundary of the editing region in the z direction relative to z=0. Therefore, a value of $\mu$ can be selected based on the desired size of editing region, where increasing the value of the centre of the boundary increases the size of the editing region (in which the weighting applied is non zero), and therefore increases the distance from the editing plane that a point will be given a non zero weighting. For example, where it is desired that the weighting applied to a point is zero if the point if further than a central plane of an adjacent slice from the editing plane (e.g. a distance of 1 from the editing plane), but that editing should otherwise be applied, a higher value of $\mu$ may be used. Generally, a value of $\mu$ which is greater than zero may be used. For very small values which essentially provide a weighting of zero (e.g. below a predetermined threshold), a weighting of zero may be applied.

Fig 6 (b) illustrates the sigmoid function as a function of z, where the influence of the smoothness parameter $\sigma$ on the sigmoid function is illustrated. In this example, values of 0.05, 0.07, 0.09, 0.11 and 0.13 (corresponding to reference numerals 642, 644, 646, 648 and 650 respectively) are set as the value of $\sigma$, and the value of $\mu$ is set as a value of zero. As can be seen from this Figure, the smaller the value of the smoothness parameter $\sigma$, the steeper the transition from 1 to 0 around the centre point (e.g. of the boundary of the editing region) will be. Therefore, by varying the smoothness parameter, the steepness of the transition between a weighting of 1 and weighting of 0 can be altered. Generally, u($\mu$) = 0.5 may be used, whereby $\mu$ is the normalized offset at which editing is applied with a 50% weight.

The sigmoid function above may therefore be considered to be the restriction function, where for a particular factor of z the restriction function provides the anisotropic weighting factor $k_{rest}$ to restrict editing in a first direction, for example above one side of the current slice (e.g. for positive z). Therefore, the anisotropic weighting factor $k_{rest}$ in a first direction z (up) can be defined as

$$k_{rest,up} = u(z). \qquad (3)$$

Values of $\mu$ and $\sigma$ may be selected so that the function value at z=0 is close to 1. As z=0 corresponds to the editing slice, it is beneficial that the weighting minimally restricts editing for points in the editing slice.

As an example, to restrict editing in a second direction, for example below the other side of the slice (e.g. for negative z), a direction opposing the first direction can be defined as -z, and therefore an equation of u(-z) may be used. Thus, we get

$$k_{rest;down} = u(-z). \qquad (4)$$

Therefore, the anisotropic weighting factor may be applied in a first direction (for example, above the slice) or a second direction (for example, below the slice). It will be appreciated that $\mu$ and/or $\sigma$ may be different for different directions (e.g. may be different for the first and second directions).

To restrict editing in both the first and second direction, the following equation may be used:

$$k_{rest} = k_{rest,up} * k_{rest,down}. \qquad (5)$$

Therefore, a two sided restriction function is provided which enables an anisotropic weighting factor to be applied to a point which lies in a first direction relative to the editing plane, and an anisotropic weighting factor to be applied to a point which lies in a second direction relative to the editing plane.

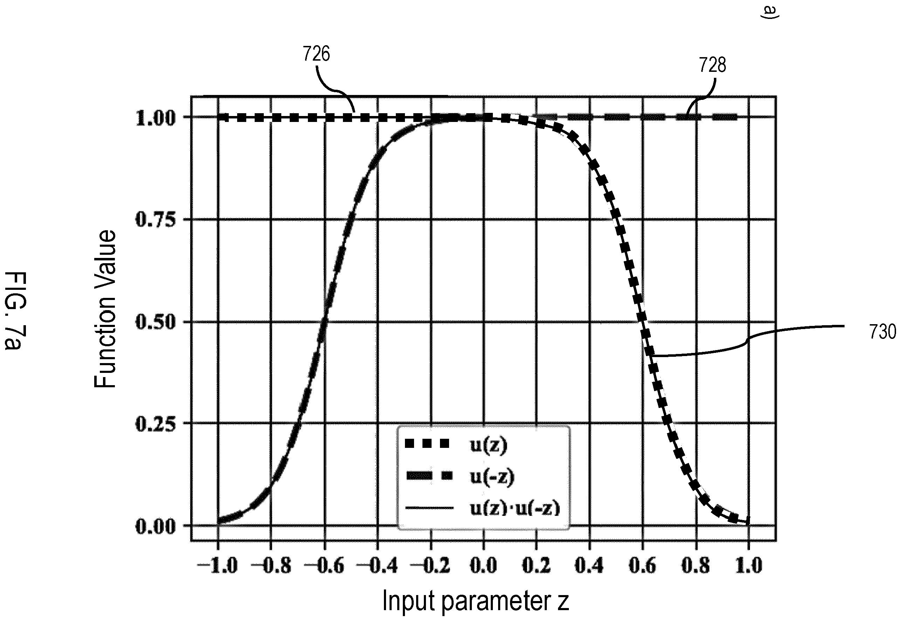

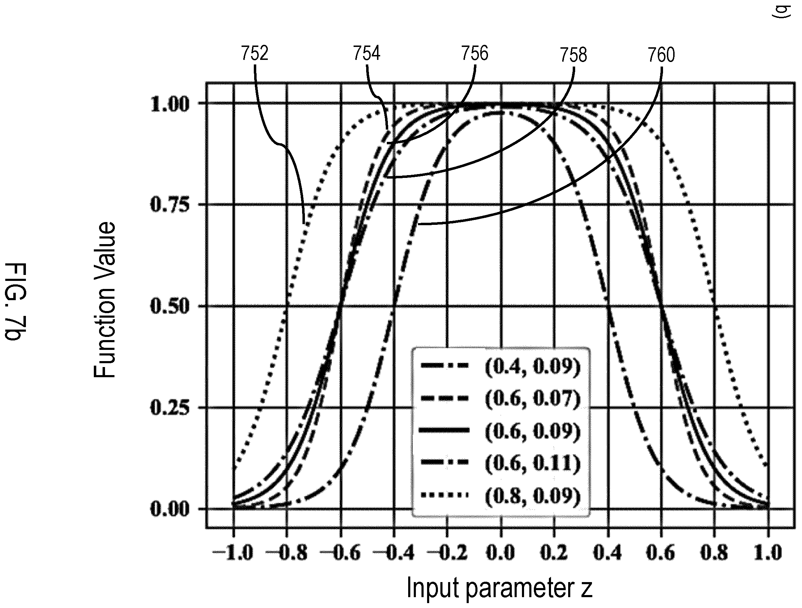

Fig. 7 illustrates single- and two-sided slice restriction functions. In particular, Fig. 7(a) illustrates u(z) 726 as a restriction function of a first direction (which in this example restricts editing over z=0 - the weight below z=0 is 1), and illustrates u(-z) 728 as a restriction function of a second direction (which in this example restricts editing below z=0 - the weight above z=0 is 1). This figure also illustrates a restriction function u(z)*u(-z) 730 which restricts editing in both the first direction and the second direction. Both single-sided functions are multiplied to form the two-sided restriction function (solid line).

Fig. 7(b) illustrates an example of a two-sided restriction function for different parameter combinations ($\mu$, $\sigma$). In particular, this Figure illustrates the restriction function for parameter combinations (0.4, 0.09), (0.6, 0.07), (0.06, 0.09), (0.6, 0.11), (0.8, 0.09) (corresponding to reference numerals 752, 754, 756, 758 and 760 respectively). As can be seen in this Figure, the steepness of the transition of the boundary of the editing region and the size of the editing region/boundary of the editing region may be selected using different values of $\mu$ and $\sigma$, where, as described above, an increase in the value of $\mu$ may increase the size of the editing region, and an increase in the value of $\sigma$ lowers the steepness of a transition in the boundary of the editing region. Values of these parameters may therefore be selected based on the requirements of the system. While this Figure illustrates a symmetrical two-sided restriction function, it will be appreciated that $\mu$ and/or $\sigma$ may be different for different directions (e.g. may be different for the first and second directions), and therefore the restriction function may be asymmetrical.

It is noted that in these examples, the boundary of the editing region is restricted so that the weighting factor reaches a value of 0 at the central plane of an adjacent slice (assuming the slices are the same width). This means that an adjacent slice in the direction that the anisotropic weighting factor is acting is not altered in the editing plane so that a user does not have to revisit previously edited slices, but a smooth transition is provided for the 3D mesh between adjacent editing planes.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer implemented method for determining editing to be applied to a three-dimensional, 3D, mesh, wherein the 3D mesh represents a segmentation of a 3D image, the method comprising:

   responsive to receiving an indication of an adjustment to be applied to a first position on the 3D mesh in an editing plane of the 3D image, adjusting a point on a boundary of the 3D mesh in an editing region of the 3D mesh using an anisotropic weighting factor that restricts the adjustment made to the point if the point lies in a first direction relative to the editing plane.

2. The method as claimed in 1, wherein the editing plane is at least one of: comprised in a slice of the 3D image; a central plane of a slice of the 3D image.

3. The method as claimed any preceding claim, wherein the first direction comprises at least one of: a direction normal to the editing plane; a direction parallel to an axis of interest; a direction parallel to an axis of an anatomical feature in the 3D image; a direction parallel to an axis of a ventricle in the 3D image; a direction parallel to an axis of a prostate in the 3D image.

4. The method as claimed in any preceding claim, wherein the anisotropic weighting factor is determined for the point based on a restriction function.

5. The method as claimed in claim 4, wherein parameters of the restriction function comprise at least one of: a distance of the point from the editing plane in the first direction; a smoothness parameter; the first direction.

6. The method as claimed in claim 5, wherein the distance of the point from the editing plane in the first direction is normalized with respect to the thickness of a slice comprising the editing plane.

7. The method as claimed in any of claims 4 to 6, wherein the restriction function is at least one of: a smooth weighting function; a sigmoid function.

8. The method as claimed in any of claims 4 to 7, wherein the restriction function restricts the editing region to define a restricted editing region.

9. The method as claimed in claim 8, wherein the restricted editing region is restricted relative to the editing region in at least one of: the first direction; the first direction and a second direction opposite to the first direction.

10. The method as claimed in any of claims 8 or 9, wherein at least one of the width and sharpness of a border of the restricted editing region in the first direction is defined relative to the thickness of a slice comprising the editing plane.

11. The method as claimed in any preceding claim, wherein anisotropic weighting factor restricts the adjustment made to the point to zero adjustment if at least one of: the point lies outside of a slice comprising the editing plane; the point lies in or beyond an editing plane of an adjacent slice.

12. The method as claimed in any preceding claim, wherein the 3D image comprises a plurality of slices to be edited in succession, and the first direction opposes a direction in which slices are to be edited.

13. The method as claimed in any preceding claim, wherein the method comprises determining an editing region based on the received indication of an adjustment to be applied to the first position on the 3D mesh, wherein the editing region is a region in which the boundary of the 3D mesh are to be adjusted.

14. A system for determining editing to be applied to a three-dimensional, 3D, mesh, wherein the 3D mesh represents a segmentation of a 3D image, the system comprising:

a memory comprising instruction data representing a set of instructions; and
a processor configured to communicate with the memory and to execute the set of instructions, wherein the set of instructions, when executed by the processor, cause the processor to:

responsive to receiving an indication of an adjustment to be applied to a first position on the 3D mesh in an editing plane of the 3D image,
adjusting a point on a boundary of the 3D mesh in an editing region of the 3D mesh using an anisotropic weighting factor that restricts the adjustment made to the point if the point lies in a first direction relative to the editing plane.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 13.

100

102

Processor

104

Memory

106

Instructions

FIG. 1

200

receiving an indication of an adjustment to be applied to a first position on the 3D mesh in an editing plane of the 3D image — 208

adjusting a point on a boundary of the 3D mesh in an editing region of the 3D mesh using an anisotropic weighting factor that restricts the adjustment made to the point if the point lies in a first direction relative to the editing plane — 210

FIG. 2

312

314

FIG. 3

FIG. 4

FIG. 5

FIG. 6a

FIG. 6b

FIG. 7a

FIG. 7b

Europäisches
Patentamt

European
Patent Office

Office européen
des brevets

# EUROPEAN SEARCH REPORT

Application Number

EP 21 18 6782

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/135305 A1 (BYSTROV DANIEL [DE] ET AL) 30 May 2013 (2013-05-30) | 1,14,15 | INV. G06T19/20 |
| Y | * abstract * * <br> * paragraph [0051] – paragraph [0052] * <br> * paragraph [0058] – paragraph [0060] * <br> ----- | 4,7 | |
| Y | EP 3 839 895 A1 (KONINKLIJKE PHILIPS NV [NL]) 23 June 2021 (2021-06-23) <br> * abstract * * <br> * paragraph [0054] – paragraph [0055] * <br> ----- | 4,7 | |

**TECHNICAL FIELDS SEARCHED (IPC)**

G06T

The present search report has been drawn up for all claims

| Place of search | Date of completion of the search | Examiner |
|---|---|---|
| Berlin | 6 December 2021 | González Arias, P |

EPO FORM 1503 03.82 (P04C01)

**ANNEX TO THE EUROPEAN SEARCH REPORT
ON EUROPEAN PATENT APPLICATION NO.**

EP 21 18 6782

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2021

| Patent document cited in search report | | Publication date | Patent family member(s) | | Publication date |
|---|---|---|---|---|---|
| US 2013135305 | A1 | 30-05-2013 | DE 212011100130 | U1 | 24-06-2013 |
| | | | US 2013135305 | A1 | 30-05-2013 |
| | | | WO 2012017375 | A2 | 09-02-2012 |
| EP 3839895 | A1 | 23-06-2021 | EP 3839895 | A1 | 23-06-2021 |
| | | | WO 2021123403 | A1 | 24-06-2021 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ECABERT, O. et al.** Automatic Model-Based Segmentation of the Heart in CT Images. *IEEE Trans. Med. Imaging,* 2008, vol. 27 (9), 1189-1201